# EUROPEAN PATENT APPLICATION

(11) **EP 2 057 939 A1**
(43) Date of publication of application: **13.05.2009**
(21) Application number: 07806030.8
(22) Date of filing: 24.08.2007
(51) Int. Cl.: A61B 3/14

(54) **OPHTHALMIC IMAGE PICKUP DEVICE**

(30) Priority: 01.09.2006 JP 2006237125; 04.09.2006 JP 2006238414; 06.09.2006 JP 2006240919; 21.08.2007 JP 2007214647
(71) Applicant: Kowa Company, Ltd., Naka-ku Nagoya-shi, Aichi-ken 460-8625 (JP)
(72) Inventor: SUZUKI, Takayoshi, Hamamatsu-shi Shizuoka 431-2103 (JP); TORIBA, Nozomu, Hamamatsu-shi Shizuoka 431-2103 (JP); MIZUNO, Takashi, Hamamatsu-shi Shizuoka 431-2103 (JP); SHIMADA, Satoshi, Hamamatsu-shi Shizuoka 431-2103 (JP)
(74) Representative: Kronthaler, Wolfgang N.K.
(86) International application number: PCT/JP2007/066412
(87) International publication number: WO 2008/029634

(57) **Abstract**

There is provided a photographic stop unit (80) comprising a fixed stop plate (31), a movable stop plate (32), and shield plates (33, 33'). A plurality of apertures (32a, 32d, 32d') is formed in the movable stop plate (32). In monocular photography, the movable stop plate moves downward, a center stop (31a) of the fixed stop plate opens, and monocular photography of the ocular fundus is performed. In stereo-photography, the movable stop plate moves upward, the center stop (31a) is closed, the shield plates (33, 33') move to the open position or the closed position in this state, the apertures (32d, 32d') for stereo-photography are alternately opened, and stereo-photography of the ocular fundus is carried out. In such a configuration, monocular photography and stereo-photography can be carried out at high speed in a simple configuration because the apertures of the photographic stop can be switched at high speed.

## Description

### Technical Field

The present invention relates to an ophthalmic photography apparatus, and more specifically to an ophthalmic photography apparatus for photographing an ocular fundus of a subject's eye as an electronic image via a photographic stop having a plurality of apertures.

### Background Art

Conventionally, there has been a need to determine the stereographic shape of the ocular fundus in order to diagnose glaucoma. Accordingly, two images that have a parallax with respect to the same subject's eye are taken as stereo-photographs and are displayed as a pair to thereby view the ocular fundus of the subject's eye stereographically.

A fundus camera that can capture an image having a parallax is provided with a photographic stop (two-aperture stop) having two left and right apertures that are positioned in conjugate with the anterior ocular segment of the subject's eye (conjugate with the pupil) with respect to the objective lens, and a beam of light that has passed through the apertures from the ocular fundus is taken as left and right images on the film surface or on the image surface of an imaging device to obtain images for stereoscopic viewing of the ocular fundus of the subject's eye.

With the configuration of the fundus camera described in the following Patent Document 1, each of two beams of light that are divided left and right by the two-aperture stop is focused separately on the film surface via light-beam dividing prisms and two separate optical systems in order to photograph the left and right images simultaneously. Also, the center distance between the two left and right apertures of the two-aperture stop is variable to make the interpupillary distance variable.

However, such a configuration as described in Patent Document 1 is only for use as stereo-photography and does not allow a switch to be made between stereo-photography and ordinary monocular photography. Also, the configuration is complicated, and the size and cost are increased because the two optical systems are provided.

Furthermore, fundus cameras are known in which photography from one aperture of a photographic stop is switched to photography from the other aperture in accordance with the operation of a shutter, and two left and right images are successively captured (Patent Document 2). Alternatively, fundus cameras are known in which a first image is captured with a single shot, a second image is then captured in a sequential manner, and the images are alternately displayed on a monitor (Patent Document 3).
Patent Document 1: Japanese Patent No.2977607
Patent Document 2: JP-A 1984-90547
Patent Document 3: JP-A 1998-75932

### Disclosure of Invention

### Problems to be solved by the Invention

For an ophthalmic photography apparatus being capable of stereo-photography, speed is required in order to instantaneously switch the left and right stop apertures during stereo-photography. However, in a conventional configuration, there is a problem in that switching between stop apertures in monocular photography and stereo-photography, as well as switching between left and right stop apertures in stereo-photography, cannot be carried out at sufficiently high speed.

The movable stop plate and the optical fiber that guides alignment-marker light for adjusting the working distance spatially interfere with each other when the movable stop plate is moved to switch the stop apertures. Therefore, there is a problem in that it is difficult to appropriately position the optical fiber in terms of the mechanism design.

There is furthermore a need to be able to change the interpupillary distance and take a stereo-photograph via a simple structure using the photographic stop mechanism.

An object of the present invention is therefore to provide an ophthalmic photography apparatus that has a simple structure and that can switch at high speed the position or the size of the apertures of the photographic stop and take a monocular photograph and a stereo-photograph of the ocular fundus without interfering with other optical elements.

### Means for solving the Problems

To solve the problems, an ophthalmic photography apparatus according to the present invention comprises:
imaging means for photographing a subject's eye as an electronic image via a photographic stop;
recording means for recording the image of the photographed subject's eye; and
a switching mechanism for moving a first movable plate and a second movable plate to switch an aperture of the photographic stop, wherein
the first movable plate is an aperture-switching plate for switching the aperture of a monocular photographic stop and the aperture of a stereo-photographic stop;
the second movable plate is a movable shield plate for opening or optically blocking off any of a plurality of apertures of the first movable plate; and
a combination of the first movable plate and the second movable plate forms a monocular photographic stop or a stereo-photographic stop.

### Advantages of the Invention

In accordance with the present invention, monocular photography and stereo-photography can be carried out at high speed using a simple configuration because the apertures of the photographic stop can be switched at high speed by moving two movable plates. A plurality of aperture pairs for individually opening two apertures of the stereo-photographic stop is formed at different distances between the two apertures in the first movable plate, and the interpupillary distance in stereo-photography can be varied using a simple configuration by selecting any of the aperture pairs. A long hole for accommodating an optical fiber for projecting alignment-marker light is formed in the movable plate along the movement direction of the movable plate. This allows the optical fiber and the movable plate to be prevented from spatially interfering with each other and alignment-marker light to be projected without obstruction.

### Brief Description of Drawings

FIG. 1 is a configuration diagram showing the overall configuration of an ophthalmic photography apparatus according to the present invention;
FIG. 2 is a chart showing the ring slit patterns that are switched in accordance with the photography mode of the ophthalmic photography apparatus;
FIG. 3 is a plan view of an apertured total reflection mirror provided to the ophthalmic photography apparatus;
FIG. 4 is a plan view showing a fixed stop plate, a movable stop plate, and shield plates that constitute the photographic stop unit of the ophthalmic photography apparatus;
FIG. 5 is an illustrative view showing the configuration of the photographic stop unit and the state of movement of the movable stop and the shield plates;
FIG. 6 is an illustrative view showing the arrangement of the apertured mirror, the fixed stop plate, the shield plate, and the movable stop plate along the photographic optical axis;
FIG. 7 is a cross-sectional view showing how a WD fiber is secured;
FIG. 8a is a flowchart showing the flow of fundus photography that is performed by selecting the photography mode;
FIG. 8b is a flowchart showing the process flow when a flare check is carried out in stereo-photography;
FIG. 8c is a flowchart that continues from FIG. 8a and shows the flow of fundus photography that is performed by selecting the photography mode;
FIG. 9 is a chart showing the relationship between the fundus camera mode, the photographic stop switching state, the on/off state of a solenoid, and the light source that emits light;
FIG. 10a is a timing chart showing the timing for observation, photography, and light emission of the light source in monocular photography;
FIG. 10b is a timing chart showing the timing for observation, photography, and light emission of the light source in stereo-photography;
FIG. 10c is a timing chart showing the timing for observation, photography, and light emission of the light source in stereo-photography when focusing is performed using focus-marker light and a flare check is carried out;
FIG. 10d is a timing chart showing the timing for observation, photography, and light emission of the light source in stereo-photography when focusing is performed based on a double image and a flare check is carried out;
FIG. 11 is a cross-sectional view showing another example of securing the WD fiber;
FIG. 12 is a plan view showing the fixed stop plate, movable stop plate, and shield plate of another embodiment of the photographic stop unit;
FIG. 13 is an illustrative view showing the configuration of the photographic stop unit and the state of movement of the movable stop and the shield plates;
FIG. 14 is an illustrative view showing the arrangement of the apertured mirror, the fixed stop plate, the shield plate, and the movable stop plate along the photographic optical axis;
FIG. 15 is a chart showing the relationship in another embodiment between the fundus camera mode, the photographic stop switching state, the movement position of the stepping motor, the on/off state of the solenoid, the light source that emits light, and the photography mode;
FIG. 16 is a block diagram showing the periphery of a control circuit for controlling the light-emission time and the light-emission time interval;
FIG. 17a is a block diagram showing a detailed configuration of a light-emission control system;
FIG. 17b is a circuit diagram showing a detailed configuration of a switching circuit in the light-emission control system;
FIG. 17c is a timing chart showing the operation of the switching circuit of the light-emission control system;
FIG. 18 is a flowchart showing the control of light emission of a flash lamp;
FIG. 19 is a chart showing the details of the light-emission time interval (t) table;
FIG. 20 is an optical view that illustrates the focusing based on the double image; and
FIG. 21 is an illustrative view showing the arrangement of the stop switch provided to the ophthalmic photography apparatus.

### Key to Symbols

- 10: Fundus camera
- 11: Halogen lamp
- 15: Flash lamp
- 21: Ring slit
- 23: Apertured total reflection mirror
- 24: Objective lens
- 26: Photographic optical axis
- 31: Fixed stop plate
- 32: Movable stop plate
- 33, 33': Shield plates
- 40, 41: CCDs
- 50: FD LED
- 54: FD light-reflecting mirror
- 66: Shutter switch
- 73: WD LED
- 75: WD fiber
- 77: Return mirror
- 80: Photographic stop unit
- 81A: to 81C Solenoids
- 82: Stepping motor
- 101: Stop switch
- 110: Flash intensity setting unit
- 111: Control circuit
- 112: Storage unit
- 113: Light-emission control system

### Best Mode of Carrying Out the Invention

Embodiments of the present invention are described in detail below with reference to the drawings. Embodiments of an ophthalmic photography apparatus are shown in which the ocular fundus of a subject's eye is photographed in the non-mydriatic mode, the mydriatic mode, and the fluorescent mode and in which ordinary monocular photography and stereo-photography are switched and carried out in the above-stated modes.

### Embodiment 1

### <Overall configuration>

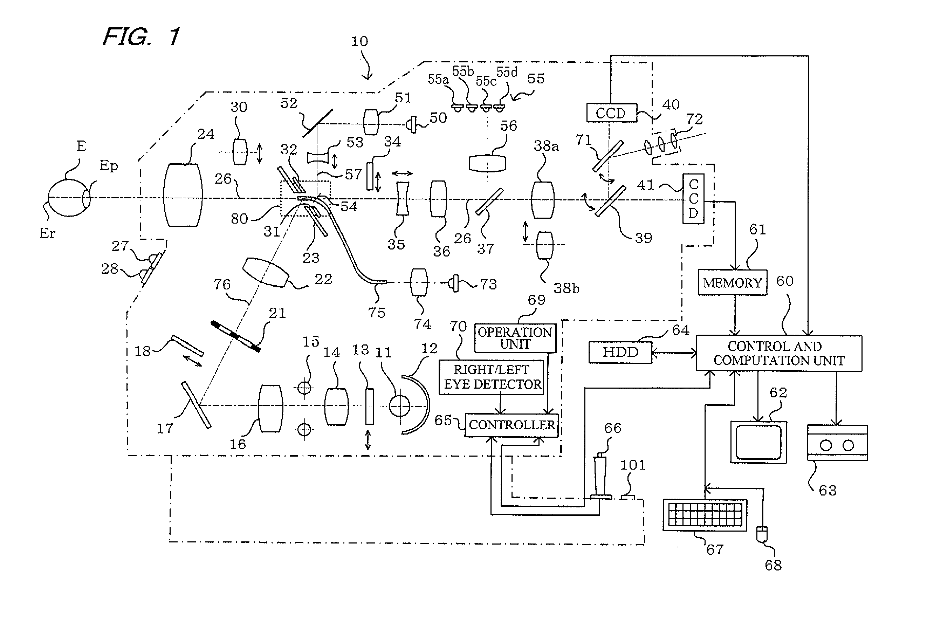

In FIG. 1, the ophthalmic photography apparatus of the present invention is composed of a fundus camera 10 for photographing the ocular fundus of the subject's eye, a memory 61 and a hard disk 64 as recording means for recording images of the photographed ocular fundus, a monitor 62 and a stereo monitor 63 for displaying images of the photographed ocular fundus or recorded images of the ocular fundus, and the like.

The fundus camera 10 enclosed and depicted by the alternate long and short dash line is provided with a halogen lamp 11 as an observation light source that emits infrared and visible illumination light and that is disposed in the center of the curvature of a spherical mirror 12. Light from the halogen lamp 11 and the spherical mirror 12 travels though a visible-blocking/infrared-transmitting filter 13 that is removably disposed in the optical path, a condenser lens 14, a flash lamp 15 (flash discharge tube) as a photographic light source, and a condenser lens 16, and is then incident on a total reflection mirror 17.

The illumination light reflected by the total reflection mirror 17 is transmitted through a relay lens 22 via a ring slit 21 used as an illumination stop, is reflected by an apertured total reflection mirror (hereinafter abbreviated as apertured mirror) 23, and is incident on an anterior ocular segment (pupil) Ep of a subject's eye E via an objective lens 24.

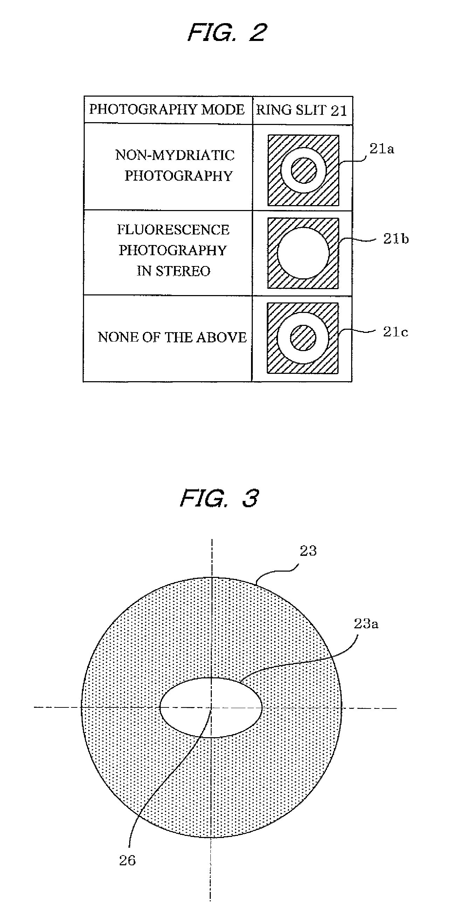

The ring slit 21 is disposed in the illumination optical system at a position substantially conjugate with the anterior ocular segment Ep (pupil) of the subject's eye, and three types of ring slits 21a, 21b, 21c are used, as shown in FIG. 2. The ring slit 21a is used in the non-mydriatic mode, the ring slit 21b is inserted into the optical path when fluorescence photography in stereo mode is performed, and the ring slit 21c is inserted into the optical path in other modes. The inside diameters of the ring slits 21a and 21c are substantially the same, the outside diameter of the slit for a non-dilated pupil is smaller, and the outside diameters of the ring slits 21b and 21c are substantially the same.

An exciter filter 18 is inserted into the optical path of the illumination optical system in fluorescence photography.

A light source 27 composed of an infrared LED (light-emitting diode) for illuminating the anterior ocular segment Ep with infrared light is provided for alignment using the anterior ocular segment, and a light source 28 composed of an LED for illuminating the anterior ocular segment with weak white light is provided in order to photograph the anterior ocular segment.

Reflected light from the ocular fundus Er illuminated by the illumination light that has passed through the ring slit 21 is transmitted through the objective lens 24, the apertured mirror 23, a fixed stop plate 31, a movable stop plate 32, a focusing lens 35, an imaging lens 36, a half mirror 37, and a variable power lens 38a, and is incident on a return mirror 39. When the return mirror 39 is positioned as shown and a return mirror 71 is rotated from the indicated position for removal from the optical path, light reflected from the ocular fundus is incident on an infrared light-sensitive CCD 40 as an imaging means that is in a position conjugate with the ocular fundus, and an image of the ocular fundus is captured by the CCD 40.

When the return mirrors 39 and 71 are positioned as shown, the light reflected from the ocular fundus is directed to an ocular lens 72. When the return mirror 39 is removed from the optical path, the light reflected from the ocular fundus is incident on a visible light-sensitive CCD 41 as an imaging means that is in a position conjugate with the ocular fundus, and an image of the ocular fundus is captured by the CCD 41.

The apertured mirror 23 is a circular total reflection mirror provided with a centrally disposed horizontally elliptical aperture 23a, as shown in FIG. 3, and is arranged so as to intersect with and slope at a predetermined angle to the photographic optical axis in a position in which the center of the aperture 23a coincides with the photographic optical axis 26.

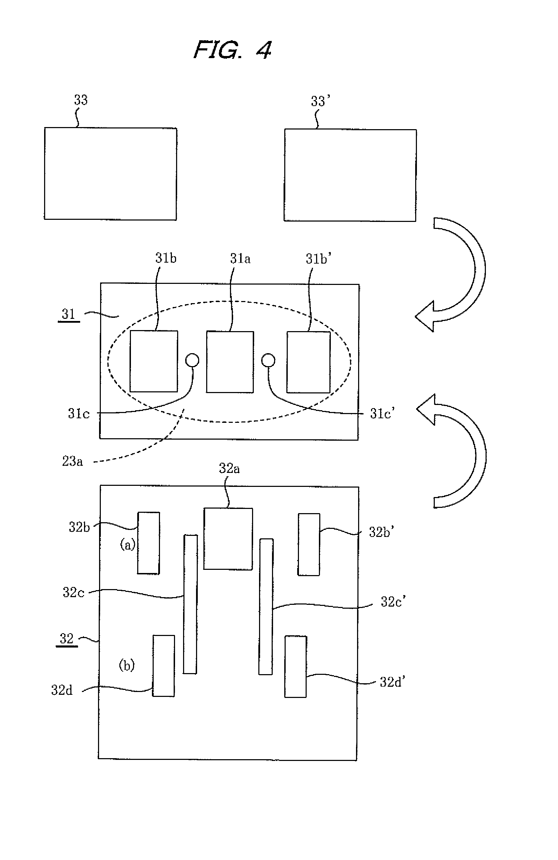

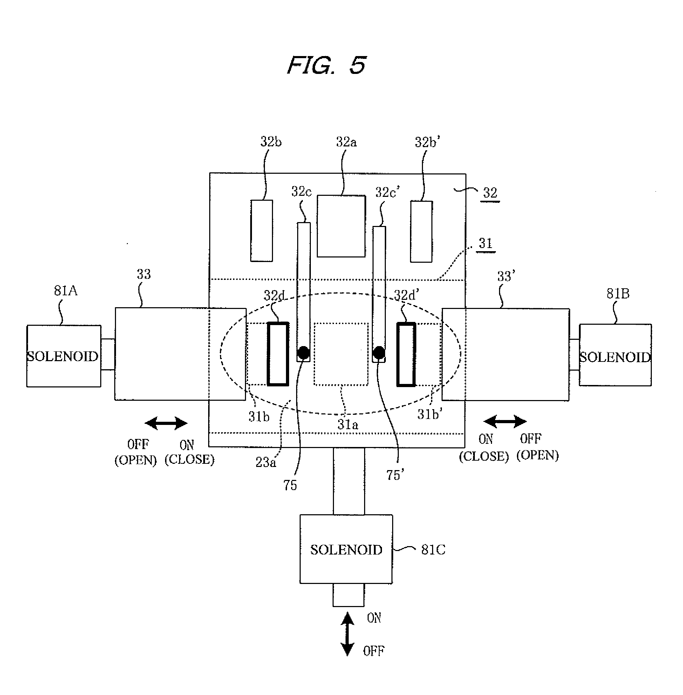

A photographic stop unit 80 is composed of the fixed stop plate (photographic stop) 31, the movable stop plate (first movable plate) 32, the left and right shield plates (second movable plates) 33, 33', and solenoids (switching mechanisms or switching means) 81A to 81C, as shown in FIGS. 4 and 5.

The fixed stop plate 31 is formed as a rectangular flat plate that is slightly larger than the aperture 23a of the apertured mirror 23, and three rectangular stop apertures 31a, 31b, 31b' (hereinafter referred to in sequence as the center stop, left stop, and right stop) are formed in a line in the center and to the left and right of the fixed stop plate 31, as shown in FIG. 4. Two apertures 31c, 31c' are formed for inserting and securing the distal ends of optical fibers (hereinafter referred to as WD fibers) 75, 75' that are used for directing alignment-marker light for adjusting the working distance (WD).

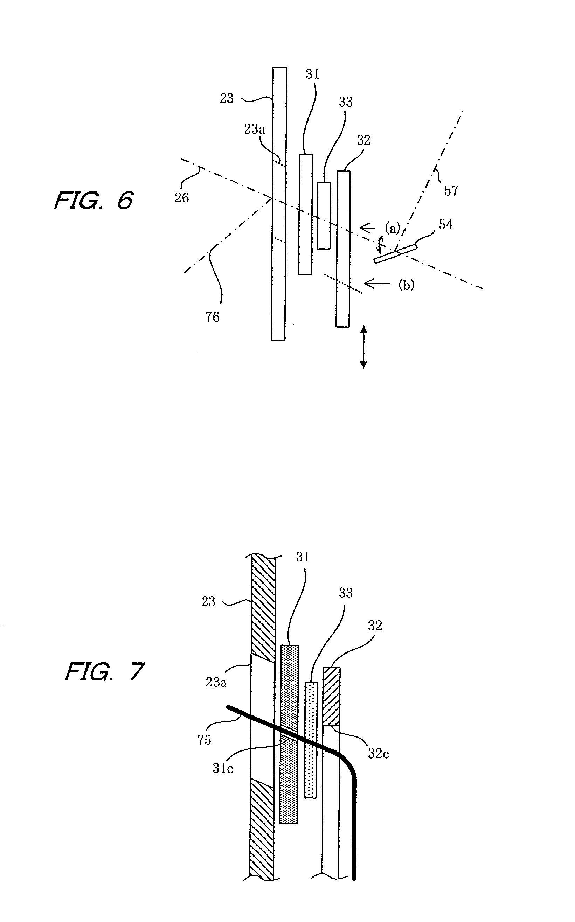

The fixed stop plate 31 is superimposed and secured (see FIGS. 6 and 7) to the reverse side (surface on the side opposite from the objective lens 24) of the apertured mirror 23, with the center (center of the center stop 31a) of the fixed stop plate in alignment with the center (center of the aperture 23a, the position of the photographic optical axis 26) of the apertured mirror 23, and is inclined and placed at the same angle as the apertured mirror 23 with respect to the photographic optical axis 26.

The stops 31a, 31b, 31b' are disposed in substantially conjugate positions with the anterior ocular segment (pupil) of the subject's eye. The center of the center stop 31a is disposed in a position that coincides with the photographic optical axis 26 (optical axis of the objective lens 24). The left stop 31b is disposed at the left optical path position obtained when the photographic optical path is divided into left and right parts at the pupil conjugate position in order to obtain left and right images for stereographic viewing. Focus-marker light (hereinafter referred to as FD light) for bringing the ocular fundus into focus passes through the left stop 31b. The right stop 31b' is disposed at the right optical path position obtained when the photographic optical path is divided into left and right parts at the pupil conjugate position, and the other beam of FD-light passes through the right stop 31b'.

The aperture 23a of the apertured mirror 23 has a size that allows substantially the entire apertures of the photographic stops 31a, 31b, and 31b' to be contained within the aperture 23a when the centers of the mirror 23 and fixed stop 31 are in alignment.

The movable stop plate 32 is formed as a rectangular flat plate that is longitudinally longer than the fixed stop plate 31, as shown in FIG. 4. The rectangular center stop 32a for observation and monocular photography is formed in the center of the upper part, and rectangular stereo-stops 32d, 32d' for stereo-photography are formed to the left and right in the lower part. Rectangular FD light windows (apertures) 32b, 32b' for passing the FD light are formed in positions offset further outside of the stereo-stops 32d, 32d' to the left and right sides of the center stop 32a. Long holes 32c, 32c' for accommodating two WD fibers are formed so as to extend in the perpendicular direction, i.e., along the movement direction of the movable stop plate, from the vicinity of the two sides of the lower half of the center stop 32a to the vicinity inside the upper half of the stereo-stops 32d, 32d'.

The center stop 32a has a size that is substantially the same as the center stop 31a of the fixed stop plate 31. The FD-light windows 32b, 32b' and the stereo-stops 32d, 32d' have a lateral width that is about half the size of the left and right stops 31b, 31b' of the fixed stop plate 31.

Here, the aperture of the center stop 32a is the aperture of the monocular photographic stop, and the stereo-stops 32d, 32d' are the apertures of the stereo-photographic stops. The movable stop plate 32 has the function of an aperture switching plate for switching the aperture of the monocular photographic stop and the apertures of the stereo-photographic stops.

The left and right shield plates 33, 33' are formed in a rectangular shape that is larger than the left and right stops 31b, 31b' of the fixed stop plate 31, respectively.

The shield plates 33, 33' are arranged so as to be superimposed on the reverse side of the fixed stop plate 31 that is superimposed on and secured to the reverse side of the apertured mirror 23 (the side opposite from the objective lens 24), as shown in FIGS. 6 and 7. The movable stop plate 32 is further arranged so as to be superimposed on the reverse side of the fixed stop plate. The order in which the shield plates 33, 33' and the movable stop plate 32 are superimposed may be reversed.

The distal end of the WD fiber 75 is inserted into the accommodation long hole 32c from the reverse side of the movable stop plate 32, is also inserted into the hole 31c of the fixed stop plate 31, and is secured in the hole 31c portion so that the distal end protrudes from the center part of the aperture 23a to the objective lens 24 side, as shown in FIG. 7. Although not depicted in FIG. 7, the distal end of another WD fiber 75' is inserted into the long hole 32c' from the reverse side of the movable stop plate 32, is also inserted into the hole 31c' of the fixed stop plate 31, and is secured at the hole 31c'. The WD fiber 75 is arranged so as to avoid interfering with the shield plates 33, 33'.

A WD LED 73 is provided as a light source for alignment-marker light (hereinafter referred to as "WD light") used to adjust the working distance (WD), as shown in FIG. 1. Light emitted from the WD LED is focused by the lens 74, directed to the rear end of the WD fiber 75, made to pass through the WD fiber 75, and projected as WD light from the distal end thereof onto the subject's eye E via the objective lens 24.

When the fixed stop plate 31, the movable stop plate 32, and the shield plates 33, 33' are arranged in a superimposed manner, the center stops 31a, 32a are aligned with each other in relation to positions in the left/right direction; the left stop 31b is aligned with the left stereo-stop 32d and the FD-light window 32b; and the right stop 31b' is aligned with the right stereo-stop 32d' and the FD-light window 32b', as shown in FIG. 5.

By switching off the solenoid 81C, the movable stop plate 32 is moved via a guide member (not shown) to a first position (hereinafter referred to as "position (a)") so that the center stop 32a may open the center stop 31a of the fixed stop plate 31, and moved by switching on the solenoid 81C to a second position (hereinafter referred to as "position (b)") for optically blocking off and closing the center stop 31a.

The solenoid 81A is switched on to move the shield plate 33 via a guide member (not shown) to a position (hereinafter referred to as the "closed position") for closing the left stop 31b and the left stereo-stop 32d or the FD-light window 32b. The solenoid 81A is switched off to move the shield plate in the opposite direction to a position (hereinafter referred to as the "open position") for opening the above-mentioned stops and window. On the other hand, the solenoid 81B is switched on to move the shield plate 33' to the closed position for closing the right stop 31b' and the right stereo-stop 32d' or the FD-light window 32b'. The solenoid 81B is switched off to move the shield plate 33' in the opposite direction to the open position for opening the above-mentioned stops and window.

FIG. 5 shows the state in which the solenoid 81C has been switched on to move the movable stop plate 32 to position (b), and the solenoids 81A, 81B have been switched off to move the two shield plates 33, 33' to the open position. In this state, the left and right stereo-stops 32d, 32d' become effective apertures. When the solenoid 81A or 81B is switched on from this state to move the shield plate 33 or 33' to a closed position, only the stereo-stop 32d or 32d' is an effective aperture.

When the solenoid 81C is switched off in the state shown in FIG. 5 to move the movable stop plate 32 to position (a), the center stop 32a and the FD-light windows 32b, 32b' become effective apertures. When the solenoids 81A and 81B are switched on from this state to move the shield plates 33 and 33' to the closed position, the FD-light windows 32b and 32b' are closed and only the center stop 32a is selected as the effective aperture of the photographic stop.

Referring back to FIG. 1, an anterior ocular segment lens 30 is removably disposed in the optical path between the objective lens 24 and the apertured mirror 23. When the anterior ocular segment lens 30 is inserted into the optical path, the image of the anterior ocular segment Ep illuminated by the illuminating light source 27 is captured by the CCD 40, and alignment is carried out using the image of the anterior ocular segment Ep.

The fundus camera is provided with a projection optical system for FD light (focus-marker light) that is used to facilitate the focusing into the ocular fundus. In the projection optical system, FD light from a FD-light LED (infrared LED) 50 as a light source passes along the optical path 57 of the FD light and through a lens 51, a mirror 52, and a lens 53. The FD light is reflected by two mirrors 54 provided to the upper part of a U-shaped member (not shown) disposed in the rearward vicinity of the photographic stop unit 80 and passes through the FD-light windows 32b, 32b' for projection onto the ocular fundus Er. The system is designed so that the image of the two focus markers that have passed through the windows are superimposed at a single point when the ocular fundus is brought into focus, while being separated when in defocus. Moving the focusing lens 35 in order to adjust the focus causes the lens 53 to move in conjunction therewith and produces a different state of separation for the FD light on the ocular fundus Er. Therefore, the examiner can obtain focus by observing the image of the focus marker.

A barrier filter 34 is inserted between the subject's eye and the focusing lens 35 during fluorescence photography.

An internal fixation lamp 55 composed of a plurality of fixation lamps 55a to 55d is provided in order to cause the subject's eye E to fixate on the fundus camera 10. One of the fixation lamps 55a through 55d is turned on depending on whether the subject's eye to be photographed is the left or right eye, and depending on the photographing position of the ocular fundus (a position near or distant from the optic nerve disk or the like). Light from the lighted fixation lamp passes through a lens 56; is reflected by the half mirror 37; passes through the photographic lens 36, the focus lens 35, the photographic stop unit 80, the apertured mirror 23, and the objective lens 24; and is projected onto the ocular fundus Er. The subject's eye E can therefore be kept at a predetermined position with respect to the fundus camera 10 by having the patient fixate on the internal fixation lamp. In the drawings, the fixation lamps 55a through 55d are shown as being placed side-by-side on the page surface. However, in actual use, the fixation lamps are placed perpendicular to the page surface.

The CCD 40 receives an image of the ocular fundus illuminated by infrared light that has passed through the visible-blocking/infrared-transmitting filter 13 during observation of the non-mydriatic eye, or an image of the anterior ocular segment illuminated by infrared light from the light source 27. The image is inputted to a control and computation unit 60, which is composed of a CPU or the like, and the resulting image is displayed as a video image on the monitor 62. The examiner can view the image displayed on the monitor 62, and perform alignment and adjust the focus. The stereo monitor 63 is provided as a dedicated display for stereoscopic viewing. The examiner can stereoscopically view the ocular fundus by observing the right and left images via the stereo monitor 63.

The CCD 41 captures a still image of the ocular fundus illuminated by the flash lamp 15 when the examiner operates a shutter switch 66. The image of the ocular fundus is temporarily stored in the high-speed memory 61 and is recorded by the control and computation unit 60 on the low-speed hard disk (HDD) 64 as an external recording device, or is displayed on the monitor 62 or stereo monitor 63.

A keyboard 67, mouse 68, or other input means is also provided, and various data can be inputted via these input devices.

A controller 65 composed of a CPU or the like is provided to the fundus camera 10. The controller 65 is connected to the control and computation unit 60 to exchange signals with each other. When the shutter switch 66 has been operated, the return mirror 39 is removed from the optical path, and the flash lamp 15 is made to emit a suitable amount of light. The controller 65 additionally controls the insertion and removal of the visible-blocking/infrared-transmitting filter 13, exciter filter 18, barrier filter 34, anterior ocular segment lens 30, and variable power lenses 38a, 38b into and from the optical path, and furthermore controls the driving of the solenoids 81A to 81C of the photographic stop unit 80.

An operation unit (operation panel) 69 is also provided to the fundus camera. The operation unit 69 has a photography mode selection switch for selecting between non-mydriatic, mydriatic, and fluorescence photography modes, as well as monocular photography and stereoscopic photography in each of these modes; a switch for inserting/removing the anterior ocular segment lens; a photographing position selection switch; and the like. Information related to the switches selected using the operation unit 69 is inputted to the controller 65.

A right/left eye detector 70 for detecting whether the subject's eye to be photographed is the left or right eye is furthermore provided, and the information about whether the detected eye is the left or right eye is inputted to the controller 65.

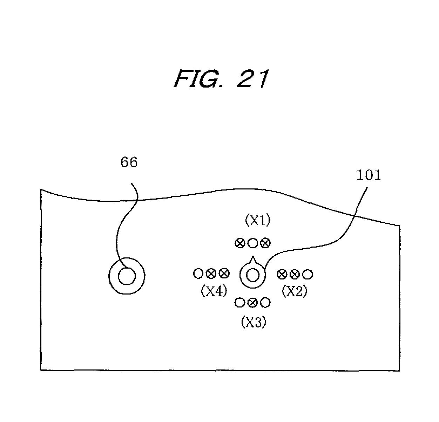

A stop switching mechanism composed of a stop switch 101 that can manually switch the movable stop plate 32 and the shield plates 33, 33' is disposed adjacent to the shutter switch 66. The arrangement is shown in detail in FIG. 21.

### <Flow of photography>

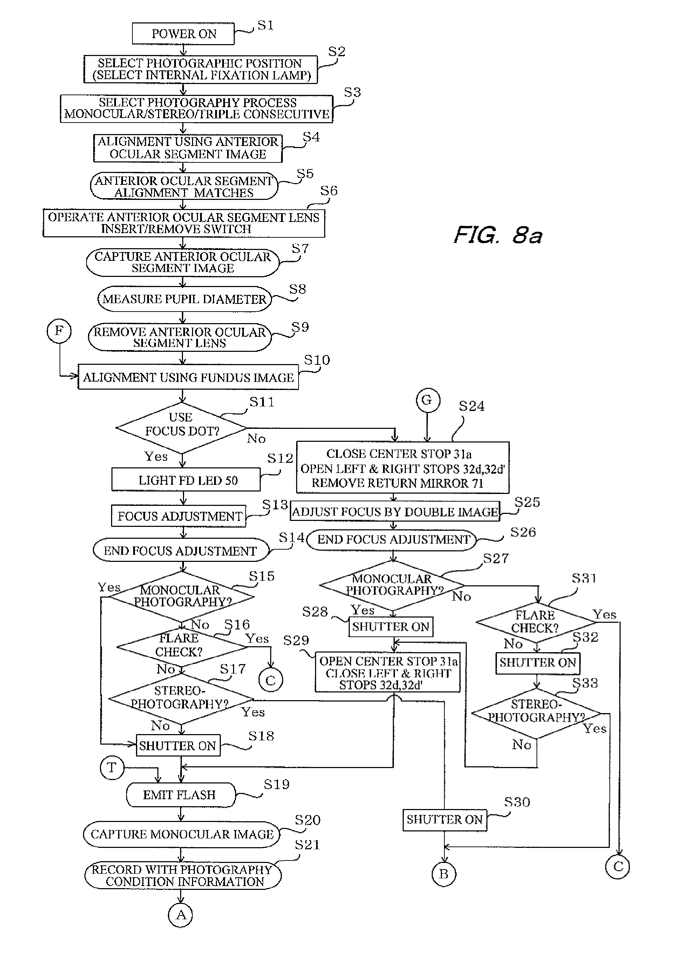

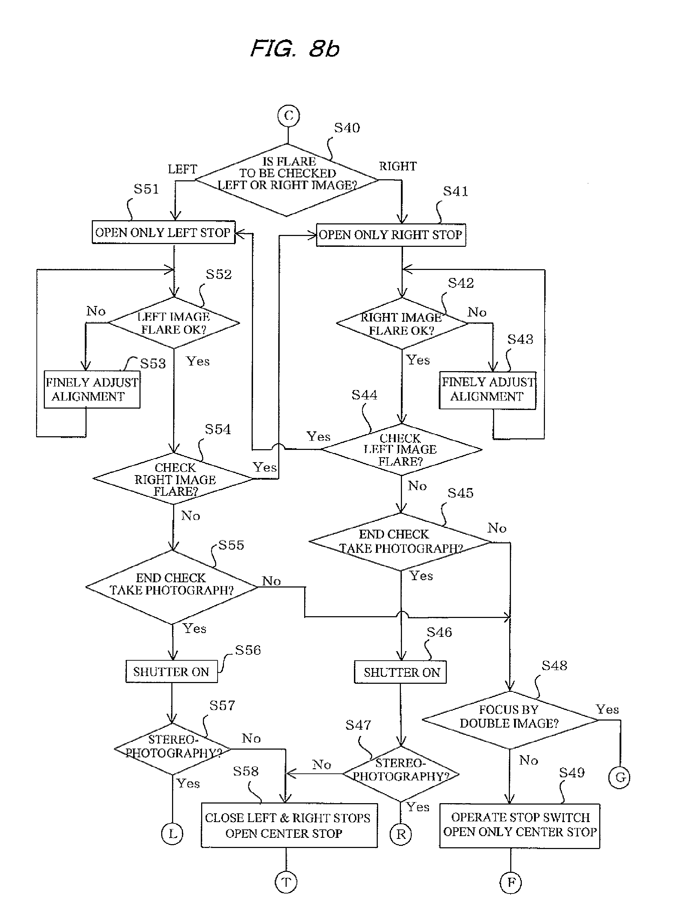

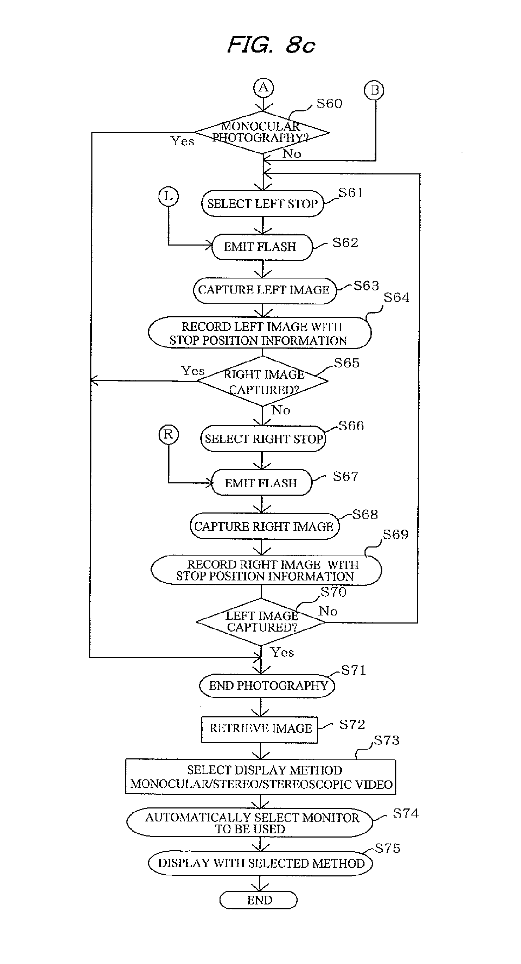

Next, the operation of the ophthalmic photography apparatus configured in the manner described above will be described in accordance with the flow of FIGS. 8a, 8b, and 8c. The operation in non-mydriatic photography mode is described below.

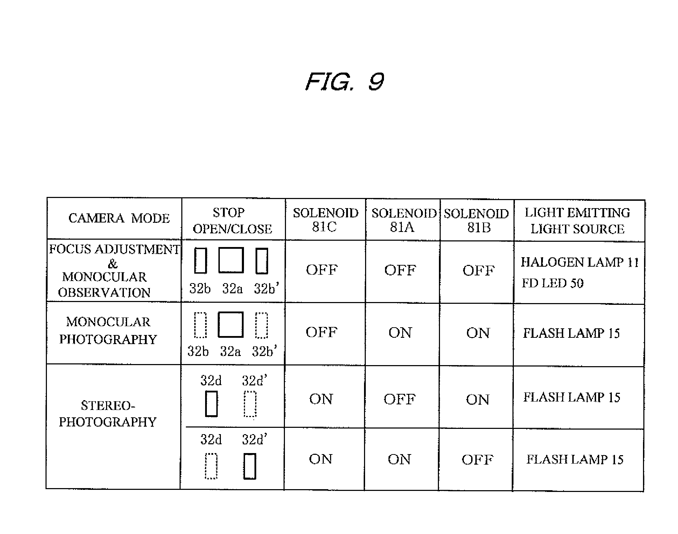

First, the open/closed state of each stop of the photographic stop unit 80 is assumed as being shown in the first row from the top of the table in FIG. 9 and set as the initial values when the power of the apparatus is switched on (step 1). In other words, all of the solenoids 81A to 81C are switched off, the movable stop plate 32 is in position (a), and the two shield plates 33, 33' are in the open position. The center stop 32a and the FD-light windows 32b, 32b' are thus selected as effective apertures. Also, the visible-blocking/infrared-transmitting filter 13 is inserted into the optical path.

The control unit 65 selects and turns on one of the fixation lamps 55a to 55d on the basis of the information from the right/left eye detector 70 and the information about the position (location) of the ocular fundus to be photographed that is selected by the photography position selection switch of the operation unit 69. The person being examined then fixates on the lighted fixation lamp (step 2).

Next, the photography mode selection switch of the operation unit 69 is used to select the monocular photography mode, the stereoscopic photography mode, or the mode in which three consecutive images are photographed (three-consecutive images photography mode), and the resulting information is inputted to the controller 65 (step S3).

The light source 27 is then turned on. The light reflected from the illuminated anterior ocular segment passes through the anterior ocular segment lens 30, and the CCD 40 captures an image of the anterior ocular segment, whose image is displayed on the monitor 62 to initiate the alignment by the anterior ocular segment (step S4). When alignment of anterior ocular segment is completed (step S5), the switch for inserting/removing the anterior ocular segment lens is operated (step S6), and a light source 28 is lighted in place of the illumination light source 27. The return mirror 39 is then removed from the optical path. Therefore, the CCD 41 captures an image of the anterior ocular segment (step S7), whose image is stored in the memory 61. The control and computation unit 60 processes the image of the anterior ocular segment stored in the memory 61 in order to calculate the pupil diameter and to detect the color of the iris (step S8).

The light source 28 is subsequently turned off and the anterior ocular segment lens 30 is removed from the optical path (step S9). Also, the halogen lamp 11 is turned on and the return mirror 39 is inserted into the optical path. The return mirror 71 is removed from the optical path.

The WD LED 73 is turned on for alignment, and WD light is projected onto the anterior ocular segment via the WD fibers 75, 75'. Light from the ocular fundus Er, which was illuminated by infrared light from the halogen lamp 11 passing through the visible-blocking/infrared-transmitting filter 13, passes through the center stop 31a of the fixed stop plate 31 and the center stop 32a of the movable stop plate 32 and is captured by the CCD 40 to form an image of the ocular fundus. The image is displayed on the monitor 62 together with the reflected image of the WD light. If the working distance is optimal, the reflected image of the WD light will be formed in a predetermined position. Therefore, the examiner performs alignment along the optical axis so that an optimal working distance may be obtained (step S10).

Focus adjustment for focusing on the ocular fundus is carried out depending upon the diopter of the subject's eye. The focus adjustment mechanism used in the present embodiment may be a focus adjustment mechanism that uses a focus dot, i.e., FD light, or a focus adjustment mechanism that uses a double image. It is therefore determined in step S11 which of the two focus adjustment mechanisms is to be used (step S11). In the case of the former, the FD LED 50 is turned on (step S12). The FD light passes through the FD-light windows 32b, 32b' of the movable stop plate 32 and through the left stop 31b and right stop 31b' of the fixed stop plate 31, and is projected onto the ocular fundus Er. The examiner operates the focusing lens 35. The lens 53 moves in conjunction with the movement of the focusing lens 35 and produces a different state of separation for the marker images on the ocular fundus. Therefore, the examiner operates the focusing lens 35 and adjusts the focus until the marker images match with each other, thereby bringing the ocular fundus into focus (step S13).

### <Monocular photography>

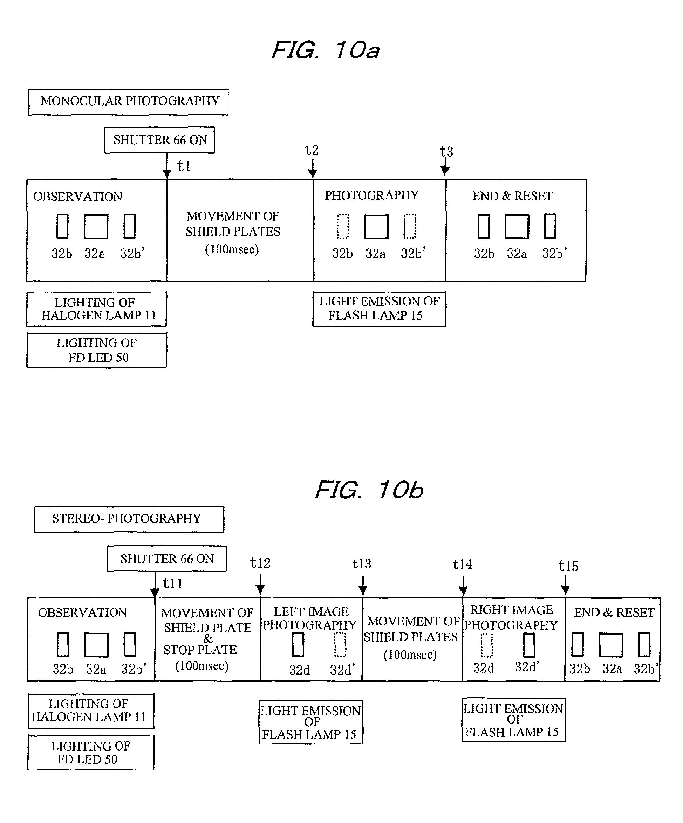

When the focus has been adjusted (step S13), a determination is made as to whether the photography mode is monocular photography (step S15). In a case in which the monocular photography mode has been selected, the process proceeds to step S18 and the shutter switch 66 is switched on. The solenoids 81A, 81B are switched on synchronously to move the shield plates 33, 33' and close the FD-light windows 32b, 32b'. The center stop 32a alone is opened to open the center stop 31a of the fixed stop plate 31. The above process is shown in FIG. 10a. The shutter switch 66 is switched on at time t1 to initiate the movement of the shield plates 33, 33'. Since the shield plates are moved by driving the solenoids 81A, 81B, the plates can be moved at high speed (e.g., about 100 msec, as shown in the diagram), and the switching of the stops from observation to photography can been performed instantaneously.

The return mirror 39 is removed from the optical path in accordance with the operation of the shutter switch 66. The flash lamp 15 emits light (step S19) at time t2 immediately following the movement of the shield plates 33, 33', and the ocular fundus is photographed via the center stop 31a of the fixed stop plate 31 and the center stop 32a of the movable stop plate 32, as shown in FIG. 10a. The ocular fundus image is temporarily recorded in the memory 61, and is thereafter processed by the control and computation unit 60 and recorded on the hard disk 64. In this case, the ocular fundus image is recorded (step S21) in association with photographic conditions such as the ID of the subject's eye, the time and date of photography, the amount of light used in the photography (amount of strobe light), an indication of the left or right eye, the position of the photographic stop, and the like.

Next, the process proceeds to the flow of FIG. 8c, and a determination is made in step S60 as to whether the photography mode is monocular photography. Since the current mode is monocular photography, the process advances to step S71 and photography is ended. The open/closed state of the stops at time t3 at the end of photography is returned to the initial state (the state during observation), as shown in FIG. 10a.

When the focus marker is difficult to use in observation, photography, and the like in the area around the ocular fundus, the focus can be adjusted via the focus adjustment mechanism using a double image without relying on a focus marker. In this case, it is determined that a focus dot will not be used during focus adjustment in step S11 of FIG. 8a, and the process proceeds to step S24. Ordinarily, the stop switch 101 is set to position X1, as shown in FIG. 21. When the stop switch is switched to position X3, the movable stop plate 32 assumes position (b). Therefore, the center stop 31a closes, the two left and right stereo-stops 32d, 32d' are set in an open state, and the return mirror 71 is removed from the optical path in conjunction with the movement of the movable stop plate 32 to position (b) (step S24).

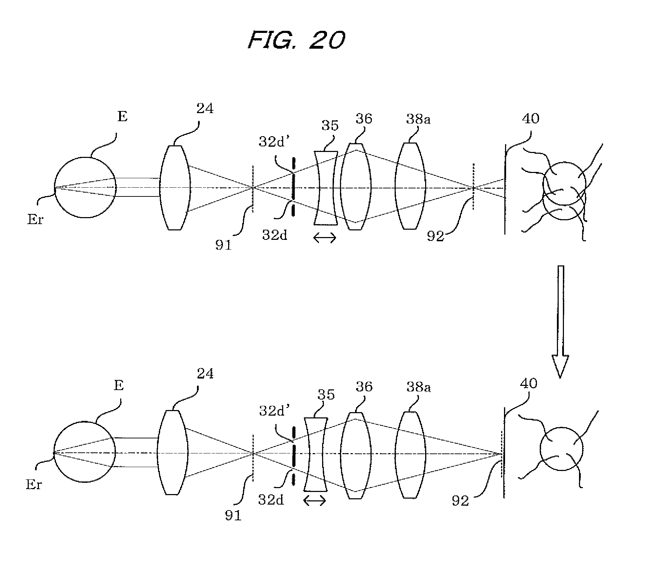

The ocular fundus is observed in this state. The image of ocular fundus Er of the subject's eye E is once formed by the objective lens 24, and passes through the left and right stereo-stops 32d, 32d' that are conjugate with the anterior ocular segment pupil. The focusing lens 35, the imaging lens 36, and the variable power lens 38a form an image on the CCD 40. When the ocular fundus is not in focus as shown in FIG. 20, an ocular fundus image 91 produced by the objective lens 24 is formed again as an ocular fundus image 92 in a position apart from the image-receiving surface of the CCD 40. Therefore, two ocular fundus images that have passed through the optical path of the stereo-stops 32d, 32d' are separated and received by the CCD 40. The two separate ocular fundus images are displayed on the monitor 62 with a portion of the images overlapping, as shown in the upper right area of FIG. 20.

The examiner then moves the focusing lens 35 in accordance with the diopter of the subject's eye and adjusts the focus. When the ocular fundus is in focus, the ocular fundus image 92 formed again by the focusing lens 35 is formed on the image-receiving surface of the CCD 40, as shown in lower part of FIG. 20. Therefore, the two ocular fundus images observed on the monitor 62 are superimposed. If the superimposition is perfect, the state is a so-called perfect focus, but if the ocular fundus images are separated and appear as a double, it is determined that the image is out of focus and the focus is adjusted until the superimposition of the images is complete (step S25).

Similarly, during observation using the ocular lens 72, the examiner visually observes the two separated ocular fundus images in a partially superimposed state when in defocus, as shown in the upper right of in FIG. 20. Adjusting the focusing lens 35 allows the two visually viewed ocular fundus images to be superimposed.

In this manner, focusing can be performed during observation of the ocular fundus prior to photography based on the ocular fundus images obtained via the two apertures of the left and right stereo-stops. Therefore, focusing can be carried out with priority for a specific location or the peripheral area of the ocular fundus because the focus state can be readily determined and there is no need to rely on a focus marker.

When focusing is completed, it is possible to proceed to photography, but in a case in which the ocular fundus is to be observed in advance, it is possible to proceed to ordinary observation using the center stop 31a because it is difficult to make an observation when unfocused locations remain in a double-image state.

When the focus has been adjusted using a double image and the process proceeds to photography, it is determined in step S27 whether the photography is monocular photography. In a case in which the monocular photography mode has been selected, the process proceeds to step S28 and the shutter switch 66 is switched on. The movable stop plate 32 moves synchronously to position (a) and the center stop 31a is opened. The solenoids 81A, 81B are switched on to close the left and right stereo-stops 32d, 32d' (step S29). The process thereafter proceeds to step S19, a flash is emitted, monocular photography is carried out in accordance with the procedure described above, and a record of the monocular photography is made (steps S20, S21, S60, and S71).

### <Stereo-photography>

On the other hand, when stereoscopic photography (stereo-photography) is selected in step S3, the process is divided into a case in which focus alignment is carried out using a focus dot to perform photography, and a case in which the focus is adjusted using a double image to perform photography. Examples are described below for both cases in which a later-described flare check is not performed.

A focus dot is used for focus adjustment (steps S11 to S13), and the focus adjustment is completed (step S14). The determinations in steps S15, S16 are negative, and the determination in step S17 is affirmative. Therefore, the shutter switch 66 is switched on in step S30. This process is shown as time t11 in FIG. 10b.

When the shutter switch 66 is switched on, the controller 65 switches the on/off states of the solenoids 81A to 81C to the state shown in the third row from the top of the chart in the table in FIG. 9, and only the left stereo-stop 32d is made an effective aperture (step S61 in FIG. 8c). The movement of the movable stop plate 32 and the shield plate 33' is also carried out at high speed, e.g., about 100 msec, in the manner shown between times t11 and t12 in FIG. 10b, thus allowing instantaneous stop switching.

Next, the flash lamp 15 emits light (step S62: time t12), and a left image that is used for stereographic viewing of the ocular fundus is formed via the left stop 31b of the fixed stop plate 31 and the stereo-stop 32d of the movable stop plate 32 (step S63). Information of the aperture position of the photographic stop when the image was captured is added to the left image, which is then recorded in the memory 61 (step S64).

Next, in a case in which the right image has not yet been captured (step S65), the controller 65 switches the on/off state of the solenoids 81A to 81C from the state shown in the third row of the chart in FIG. 9 to the state shown in the fourth row, moves the shield plates 33, 33' to the closed and open positions (time t13 of FIG. 10b), respectively, and opens the right stereo-stop 32d' (step S66: time t14). The movement of the shield plates 33, 33' is also carried out at high speed, e.g., instantaneously at about 100 msec, in the manner shown between times t13 and t14 of FIG. 10b.

Immediately after the movement of the shield plates 33, 33', the flash lamp 15 automatically emits light (step S67: time t14), and the right image for stereographic viewing is photographed via the right stop 31b' of the fixed stop plate 31 and the right stereo-stop 32d' of the movable stop plate 32 (step S68). Similarly to the left image, information about the aperture position of the photographic stop during image capture is added to right image, which is then recorded in the memory 61 (step S69). After the right stereo-photographic image has been captured, the open/closed state of the photographic stop is returned to the initial state, and stereo-photography is ended (step S71: time t15).

In this manner, the two stereo-photography apertures (31b, 31b'; and 32d, 32d') of the photographic stop are automatically switched using a single shutter operation in stereo-photography, and the flash lamp 15 automatically emits light in synchronization with the switching of the apertures. Therefore, the left and right images for stereographic viewing can be captured in a single shutter operation.

### <Stereo-photography with flare check>

In the stereo-photography described above, unpredictable flare enters the photographed image and results in photography failures because the apertures used during observation and those used during photography are often different .

There is thus a need to check flare in advance when still stereo-photography is carried out at the end of the observation. A procedure is therefore adopted in which the stop switch 101 is operated, the center stop is closed, and only one of the left and right apertures is opened to allow the ocular fundus and flare to be checked through the same stop aperture that will be used during photography. Therefore, in a case in which the flare check is to be made prior to stereo-photography in FIG. 8a (yes in steps S16, S31), the flare is checked to perform photography.

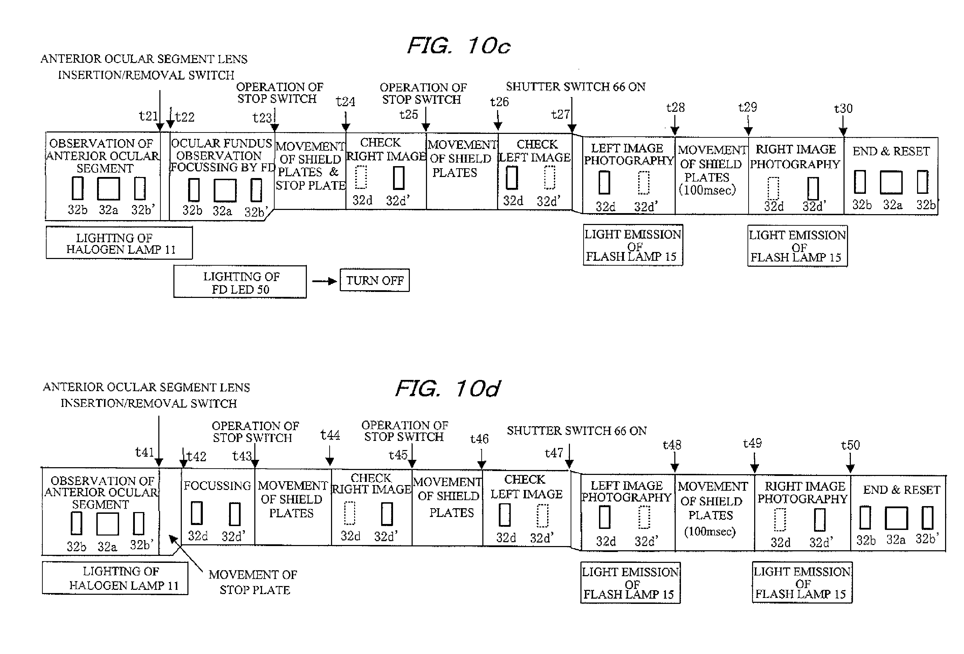

The flow of stereo-photography that includes a flare check is shown in FIGS. 10c, 10d, and 8b. FIG. 10c shows the flow in a case in which focus adjustment is performed using FD light and a flare check is performed for stereo-photography. FIG. 10d shows the flow in a case in which focus adjustment is performed using a double image and a flare check is performed for stereo-photography.

First, in a case in which focus adjustment is performed using FD light, the anterior ocular segment lens is removed from the optical path using the switch for inserting/removing the anterior ocular segment lens at time t21, as shown in FIG. 10c. The FD LED 50 is lighted at time t22 (step S12 in FIG. 8a), and focus adjustment is performed using FD light (step S13). After the focus has been adjusted (step S14), a flare check is affirmed (step S16), and the process proceeds to the flow of FIG. 8b.

In FIG. 8b, it is determined whether the flare is checked for the left or right image for stereographic viewing (step S40). In the case of the right image, the stop switch 101 is set to position X2 (FIG. 21) at time t23. The movable stop plate 32 is then moved to position (b), the shield plate 33 is moved to the closed position, and the shield plate 33' is moved to the open position. Therefore, only the right stereo-stop 32d' opens (step S41) and the right image can be observed. A check is then made as to whether flare has occurred in the right image (step S42). The timing of this procedure is shown at t24 of FIG. 10c. The FD LED 50 is turned off at this point.

If flare is found in the right image by ocular fundus observation via the right stereo-stop 32d', it shows that the alignment is inappropriate. Therefore, the alignment is finely adjusted (step S43). On the other hand, when there is no flare, a determination is made as to whether flare should be checked in the left image as well (step S44). When a flare check is to be performed for the left image, the stop switch 101 is set to position X4 (FIG. 21) at time t25. This causes the shield plate 33 to be moved to the open position and the shield plate 33' to be moved to the closed position. Therefore, only the left stereo-stop 32d is opened (step S51) and the left image can be observed. A determination is then made as to whether there is flare in the left image (step S52: time t26). When flare has been confirmed, alignment is finely adjusted (steps S52, S53)

In this manner, the image flare is checked by ocular fundus observation using one stop. If there is no problem, the stop switch 101 is operated again to invert the state of the stop aperture, and the flare state is checked using the other stop.

After it has been confirmed that flare is not present in the left image, the shutter switch 66 is switched on (step S56) in a case in which the left image is to be photographed (step S55). The process proceeds from step S57 to step S62 of FIG. 8c, and a flash is emitted. The left image is thus photographed and recorded (steps S63, S64: time t27 in FIG. 10c)

On the other hand, after it has been confirmed that flare is not present in the right image, the shutter switch 66 is switched on (step S46) in a case in which the right image is to be photographed (step S45). Since stereo-photography has been selected, the process proceeds to step S67 of FIG. 8c, and a flash is emitted. The right image is photographed and recorded (steps S68, S69). The flow of left image photography and right image photography is shown in FIG. 10c from time t27 to t30, and the flow is the same as from time t12 to time t15 in FIG. 10b.

During flare check, the double-image state or monocular state can be reinstated as required. The aperture state of the stereo-stops can be suitably switched at any time and any number of times depending upon the intentions of the examiner. For example, the process proceeds to step S24 of FIG. 8a in a case in which focus adjustment is performed using a double image (yes in step S48). It is also possible to operate the stop switch 101, open only the center stop 31a (step S49), proceed to step S10 of FIG. 8a, and observe the ocular fundus for alignment. When stereo-photography is not carried out in steps S47, S57, it is possible to close the left and right stereo-stops 32d, 32d', open the center stop 31a (step S58), proceed to step S19 of FIG. 8a, and perform monocular photography.

The example described above is one in which the focus is adjusted using FD light and stereo-photography is performed after a flare check. FIG. 10d also shows the flow of a case in which focusing is carried out using a double image and stereo-photography is carried out after a flare check.

In a case in which the focus is adjusted using a double image, the anterior ocular segment lens is removed from the optical path using the switch for inserting/removing the anterior ocular segment lens at time t41, as shown in FIG. 10d, and the focus is adjusted using a double image at time t42 (step S25 of FIG. 8a). After focus adjustment has been completed (step S26), the flare check is affirmed (step S31), the process proceeds to the flow of FIG. 8b, and the above-described process is carried out. The flow from time t43 to t50 of FIG. 10d corresponds to the flow from time t23 to t30 in FIG. 10c.

In the example described above, the right image is photographed after the left image has been photographed, but photography is carried out using the same flow also when the order is reversed.

In this manner, the single operation of the shutter switch 66 causes only one of the stereo-stop apertures on the right or left side to be opened to take one of the still images for stereographic viewing. The opened aperture is subsequently closed, and the other aperture that had been closed up to that point is opened to take the other still image for stereographic viewing. It is preferred in terms of apparatus operation that still image photography for stereographic viewing be started using the opened one of stereo-stop apertures when it is used immediately before the shutter switch has been pressed. When double images are being observed or when the center stop 31a is being used immediately before the shutter switch is pressed, the two apertures (32d and 32d') of the stereo-stops are sequentially opened in a preset sequence to perform stereo-photography upon operation of the shutter switch.

### <Photography mode for three consecutive images>

If, in step S3, the photography mode for three consecutive images has been selected, monocular photography is followed by stereo-photography without interruption. Monocular photography is ended at time t3 in FIG. 10a, after which the process proceeds to time t11 of FIG. 10b and the process advances to aperture switching for stereo-photography to perform stereo-photography (time t12 to t15). A single shutter operation causes the flash lamp 15 to emit light three consecutive times. A monocular image is formed using the first light emission, the left image for stereographic viewing is photographed using the second light emission, and the right image for stereographic viewing is photographed using the third light emission.

In the embodiment described above, the non-mydriatic photography mode was described, but a mydriatic photography mode and a fluorescence photography mode may also be used. In the mydriatic photography mode, the visible-blocking/infrared-transmitting filter 13 is removed from the optical path, the return mirror 71 is inserted into the optical path, and observation is performed via the ocular lens 72. Also, in the case of fluorescence photography, the exciter filter 18 and the barrier filter 34 are inserted into the optical path to carry out photography.

In the embodiment described above, the photographed images are temporarily recorded in the memory 61, and the images recorded in the memory 61 are transferred to the external hard disk 64 with predetermined timing. In this case, the timing with which the images recorded in the memory 61 are transferred to the hard disk 64 is varied depending upon whether or not monocular photography is performed. For example, except in monocular photography, the images are saved in the memory 61 until a predetermined number of images has been taken, and are thereafter transferred to the external hard disk 64. In fluorescence photography, the images are converted to black and white images by the control and computation unit 60, and the converted images are saved on the external hard disk 64.

When an image recorded in the memory 61 or the external hard disk 64 is retrieved and displayed (step S72 of FIG. 8c), the display method and display means (monitor) can be varied in accordance with the photography mode (steps S73 to S75). For example, when the ocular fundus image photographed in monocular photography is to be displayed, the monitor 62 is automatically selected, and the ocular fundus image is displayed together with the information of the photography conditions as a still image on the monitor 62. Also, when two left and right images obtained in stereo-photography are retrieved and the ocular fundus is stereographically viewed, the stereo monitor 63 is used. The image with the information of the left position is displayed on the left side, the image with the information of the right position is aligned on the right side, and other information about the photography conditions is also displayed.

In the present embodiment, the apertures of the photographic stops can be instantaneously switched at high speed. Therefore, myosis, position displacement, and other problems can be reduced in, e.g., stereo-photography in which the two left and right ocular fundus images for stereographic viewing are photographed, thus allowing good stereo-photography to be carried out.

The distal ends of the two WD fibers 75, 75' are inserted into, respectively, the long holes 32c, 32c' formed so as to extend along the perpendicular direction, which is the movement direction of the movable stop plate 32, and are secured to the fixed stop plate 31. Therefore, the index markers produced by the WD fibers can be projected without obstruction and without interfering with the movement of the movable stop plate 32.

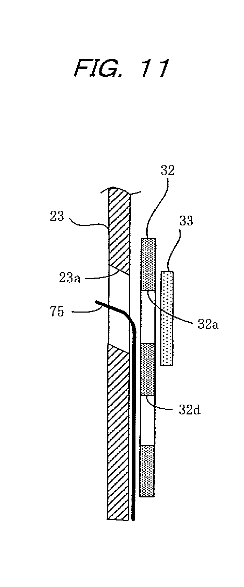

The WD fiber 75 may be arranged so that the curved distal end of the WD fiber 75 is secured to the reverse surface of the apertured mirror 23, as shown in FIG. 11, and so that the distal end protrudes only slightly from the center of the aperture 23a. In such a configuration, the fixed stop plate 31 is not needed, and a long hole for accommodating the WD fiber is also not required in the movable stop plate 32.

### <Flash intensity adjustment>

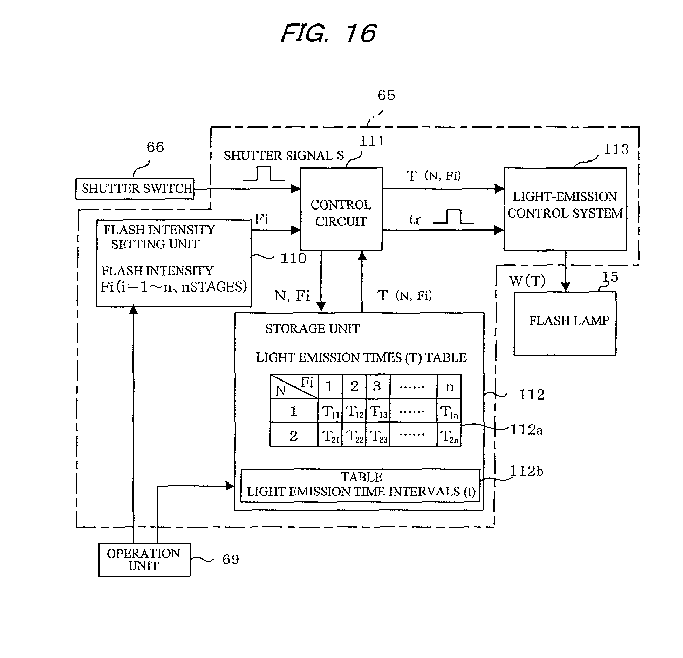

In the embodiment described above, it is preferred that the two captured images for stereographic viewing have substantially the same brightness when stereo-photography is carried out. Accordingly, a mechanism for adjusting the intensity of the flash that can make small adjustments in terms of intensity is disposed inside the controller 65 so that the brightness of the image obtained in the first photography is kept substantially the same as the brightness of the image obtained in the second photography. FIG. 16 shows such a mechanism for adjusting the intensity of the flash.

In FIG. 16, the controller 65 is provided with a flash intensity setting unit 110, a control circuit 111, a storage unit 112, and a light-emission control system 113. The flash intensity setting unit 110 sets the flash intensity Fi via a variable resistor, a rotary switch, or the like that is provided to the operation unit 69 operated by the user. The flash intensity Fi may be set to n stages Fi to Fn, for example.

The control circuit 111 is composed of a CPU, a ROM (not shown) in which later-described programs are stored, and the like. A shutter signal S from the shutter switch 66 and the flash intensity Fi from the flash intensity setting unit 110 are inputted to the control circuit 111 via an input circuit such as an I/O port.

In the present embodiment, the amount of light emitted by the flash lamp 15 is controlled using the light-emission time T, and the storage unit 112 is provided with a table 112a for storing the light-emission time T that depends on the number of light emissions N and the flash intensity Fi that has been set in the flash intensity setting unit 110. Here, the number of light emissions N is determined by the photography mode selected in step S3 of FIG. 8a.

The control circuit 111 receives the shutter signal S and the flash intensity Fi from the flash intensity setting unit 110, reads from the table 112a the light-emission time T (N, Fi) that depends on the number of light emissions N and the flash intensity Fi, and outputs the result to the light-emission control system 113. When, for example, two consecutive light emissions are performed in stereo-photography with the flash intensity Fi set to 2 in the table as shown in the diagram, T12 is outputted to the light-emission control system 113 as the light emission time T (N, Fi) in the first light emission, and T22 is outputted as the emission time T (N, Fi) in the second light emission.

It is apparent that the setting values in the table 112a stored in the storage unit 112 are determined based on actual measurement results or the like obtained in advance. Accordingly, the stored values are values that are determined in accordance with the actual photography intervals and the characteristics of the light-emission control system 113.

The control circuit 111 generates a light-emission trigger signal tr at predetermined timing when a shutter signal S is generated. The light-emission control system 113 receives the trigger signal tr and causes light to be emitted by sending power to the flash lamp 15 for a length of time that is equal to the light-emission time T described above.

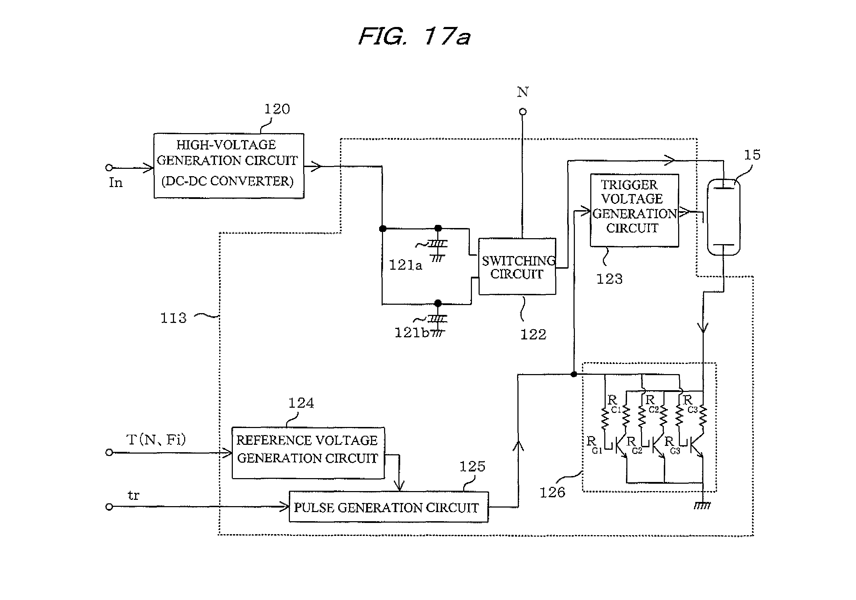

FIG. 17a shows a detailed configuration of the light-emission control system 113. The light-emission control system 113 has a plurality of capacitors 121a, 121b for causing the flash lamp 15 to emit light, and each of the capacitors is charged by a high-voltage generation circuit 120 composed of a DC/DC converter or the like connected to a power source input In. A switching circuit 122 determines which of the capacitors 121a, 121b is used to cause the flash lamp 15 to emit light.

The switching circuit 122 is switched by the number of light emissions N provided from the control circuit 111. Alternatively, the light-emission control system 113 may be provided with a light-emission counter that counts the number of light emissions and produces the counted output N. The switching circuit 122 connects one of the capacitors 121a, 121b to the flash lamp 15 in accordance with the number of light emissions.

The light-emission time T (N, Fi) described above and the light-emission trigger signal tr are inputted to a reference voltage generation circuit 124 of the light-emission control system 113 and a pulse generation circuit 125, respectively.

The reference voltage generator 124 generates a reference voltage for controlling the pulse generation circuit 125 on the basis of the inputted light-emission time T (N, Fi), and the pulse generation circuit 125 determines the energizing time of the flash lamp 15 on the basis of the reference voltage inputted from the reference voltage generating circuit 124.

The pulse generation circuit 125 triggers the flash lamp 15 via a trigger voltage generation circuit 123 at the timing of the inputted light-emission trigger signal tr, and simultaneously switches on the ground side of the flash lamp 15 via a transistor circuit 126 using insulation gate-type bipolar transistors. Power to the flash lamp 15 is cut off via the transistor circuit 126 following a light-emission time that corresponds to the reference voltage inputted from the reference voltage generator 124.

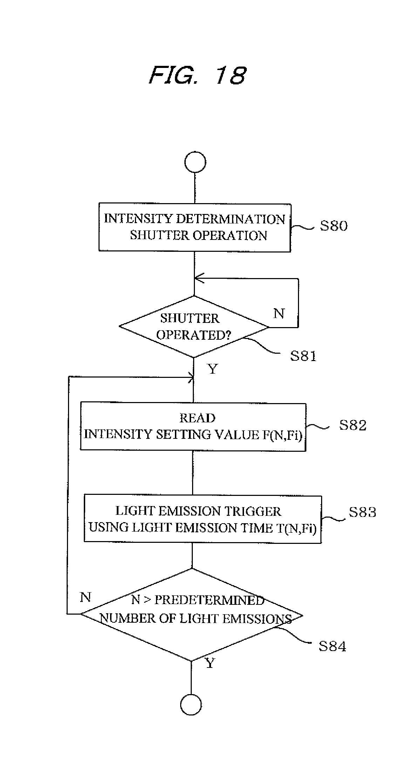

The light-emission control procedure controlled by the control circuit 111 has a flow such as that shown in FIG. 18. For example, the control procedure of FIG. 18 can be stored in a ROM or other storage means as a program executed by the control circuit 111.

In FIG. 18, the user (examiner) operates a rotary switch of the operation unit 69, sets a desired flash intensity Fi via the flash intensity setting unit 110, and operates the shutter switch 66 (step S80). The operation is detected (step S81) by the control circuit 111, and the control circuit 111 accordingly reads the flash intensity Fi that has been set in the flash intensity setting unit 110 (step S82).

The number of light emissions N is determined by the photography mode selected in step S3 of FIG. 8a. In step S83, the light emission time T(N, Fi) is read from the table 112a of the storage unit 112 in accordance with the flash intensity Fi and the number of light emissions N (the value indicating the ordinal number of the light emission that will be performed next), and is outputted together with the trigger signal tr to the light-emission control system 113. The flash lamp 15 is made to emit light in accordance with the provided light emission time T(N, Fi). As a result, the light-emission control system 113 provides the flash lamp 15 with a drive power W(N, Fi) that is determined in accordance with the provided light emission time T(N, Fi).

In step S84, it is determined whether the light emission having the number of light emissions N set in accordance with the photography mode has ended. In the case of stereo-photography, N = 2, and the process ends when the light emissions having the number of light emissions N have ended, otherwise the process is repeated from step S82.

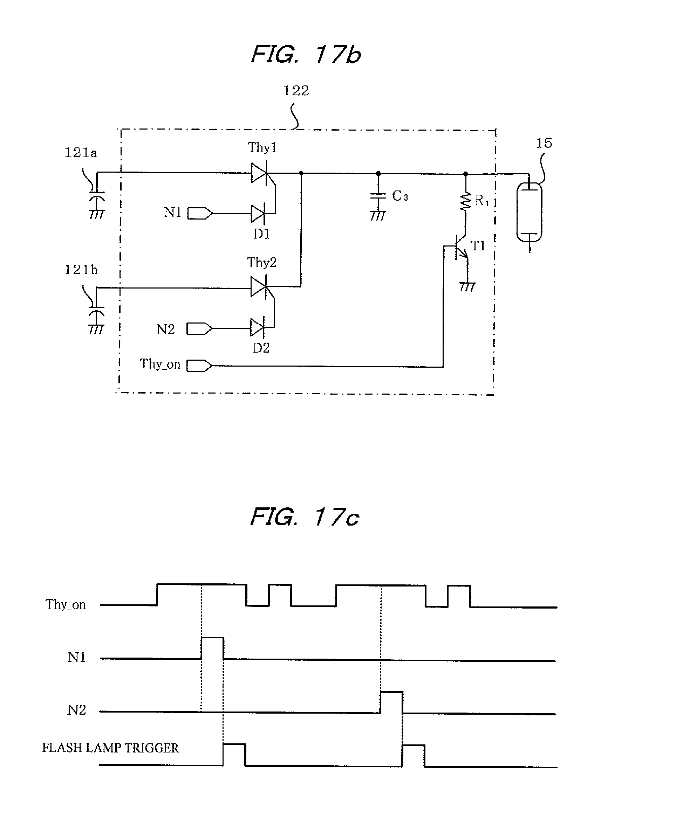

In the light-emission control system 113 shown in FIG. 17a, the two capacitors 121a, 121b are switched to perform stereo-photography. In this configuration, light emission and charging are carried out independently by each of the capacitors. This allows consecutive (stereo) photography to be carried out with short light emission intervals (100 msec) even in such cases as fluorescence photography in which the light emission intensity is greater. Since the switching of the capacitors is carried out by the switching of a high-voltage circuit, a switching circuit 122 is provided that uses thyristors and transistors as shown in FIG. 17b.

In FIG. 17b, the capacitors 121a, 121b are switched in accordance with the light emission number signals N1, N2 by a thyristor-on signal (Thy_on) as shown in FIG. 17c. Power is applied to the flash lamp 15 to cause the flash lamp 15 to emit light.

In this manner, when stereo-photography is to be performed, the flash intensity Fi is set, the first and second light emission times that have been preset according to the light emission timetable 112a are read, and the flash lamp is energized. This allows the flash intensity to be adjusted so that the brightness of the image obtained in the first photography is substantially the same as that of the image obtained in the second photography.

### <Adjustment of the photography interval>

In the case of performing consecutive photography in which consecutive still images are captured in various photography modes, it is sometimes difficult to constantly emit light and take photographs at 100-ms intervals such is the case with the non-mydriatic mode. Accordingly, the time interval from the first photography to the second photography must be optimized. For this reason, the time interval from the first photography to the second photography can be automatically set in accordance with the set photography mode and/or the selected photography mode.

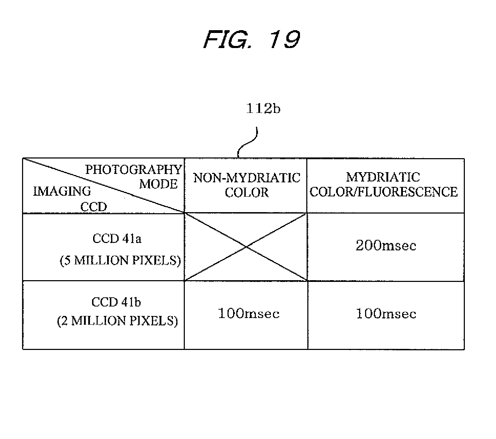

In other words, a table 112b of light emission time intervals (t) such as that shown in FIG. 19 is provided to the storage unit 112 in FIG. 16. Data on the light emission time interval (t) is searched from the table 112b of light emission time intervals (t) in accordance with the photography conditions (photography mode/imaging CCD) set in the operation unit 69 in stereo-photography. When the shutter signal S produced by the operation (on) of the shutter switch 66 is inputted to the control circuit 111, the control circuit 111 sets the searched light emission time interval (t) as a time interval from the first photography to the second photography (hereinafter referred to as "consecutive-shot time interval"), and outputs the trigger signal tr to the light-emission control system 113 at this time interval. The light-emission control system 113 feeds power for light emission to the flash lamp 15 two times in synchronization with the trigger signal tr. This causes the flash lamp 15 to emit light in stereo-photography twice at the light emission time interval (t) and the two left and right ocular fundus images to be consecutively shot at the light emission time interval (t).

The light emission time interval (t) is varied in accordance with the imaging means and/or the photography mode to be used. Following are the reasons for varying the light emission time interval using imaging means.

An imaging CCD having a high pixel count inevitably needs a grater data transfer time, and a lower pixel count results in a shorter transfer time. When the transfer time is great, the consecutive-shot time interval must be extended. For example, when the number of pixels is 2 million, a consecutive-shot time interval of about 100 msec is sufficient, but when the number of pixels is 5 million, about 200 msec is required. Therefore, the minimum required consecutive-shot time interval varies depending on whether the imaging CCD 41 is a high-resolution CCD 41a of 5 million pixels or a lower resolution CCD 41b of 2 million pixels.

The consecutive-shot time interval must also be varied depending on the photography mode. For example, when the photography mode is non-mydriatic color, the consecutive-shot time interval must be set to about 100 msec because the second photograph must be taken before the pupil of the subject's eye is contracted by the photography light emitted into the subject's eye in the first photograph.

However, in the mydriatic mode (including the fluorescence mode) in which a mydriatic agent is dropped into the subject's eye for photography, the limitation of the above-described pupil contraction is not present and the consecutive-shot time interval is not required to be limited to about 100 msec. Additionally, a high-resolution CCD is preferred because a highly detailed image is required in fluorescence photography and mydriatic photography. Therefore, it is possible to consider carrying out photography using a 5-million pixel CCD, even when the consecutive-shot time interval is, e.g., 200 msec.

In the case of the non-mydriatic color mode, the consecutive-shot time interval must be set to 100 msec. Therefore, the CCD 41a, which requires a greater data transfer time, cannot be used, and the CCD 41b must unavoidably be used.

The light emission time (T) and/or the light emission time interval (t) of the photographic light is thus set by the controller 65 in accordance with the photography mode set in the operation unit 69, the photographic light intensity, and the selected imaging means. This setting makes it possible to carry out the first and second photographs in stereo photography and provide two good ocular fundus images for stereographic viewing.

### Embodiment 2

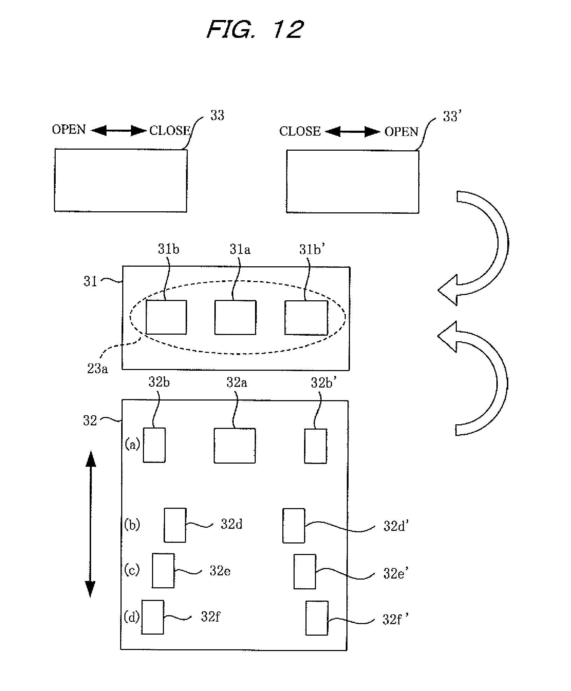

Next, another embodiment will be described with reference to FIGS. 12 to 15. In the drawings, the same reference numerals are used for the same or corresponding portions in FIGS. 1 to 11, and a description of the same portions will be omitted.

The center stop 32a and the FD-light windows 32b, 32b' are formed in a line in position (a) of the upper portion in the perpendicular direction in the movable stop plate 32, as shown in FIG. 12, and three pairs of left and right stereo-stops 32d and 32d', 32e and 32e', and 32f and 32f' are formed in positions (b), (c), and (d) below position (a). The positions in the left and right directions of the left stereo-stops 32d, 32e, 32f are positions superimposed on the left stop 31b of the fixed stop plate 31, and the positions in the left and right directions of the right stereo-stops 32d', 32e', 32f' are positions superimposed on the right stop 31b'.

Also, the stereo-stops 32d and 32d', 32e and 32e', and 32f and 32f' correspond in sequence to small parallax stereo-photography, medium parallax stereo-photography, and large parallax stereo-photography. The distance between the apertures increases in the stated order, corresponding to the distances between the pupils of e.g., 2 mm, 3 mm, and 4 mm, respectively.

Although not depicted, two long holes for accommodating the WD fiber are formed in the movable stop plate 32 in the same manner as Embodiment 1.

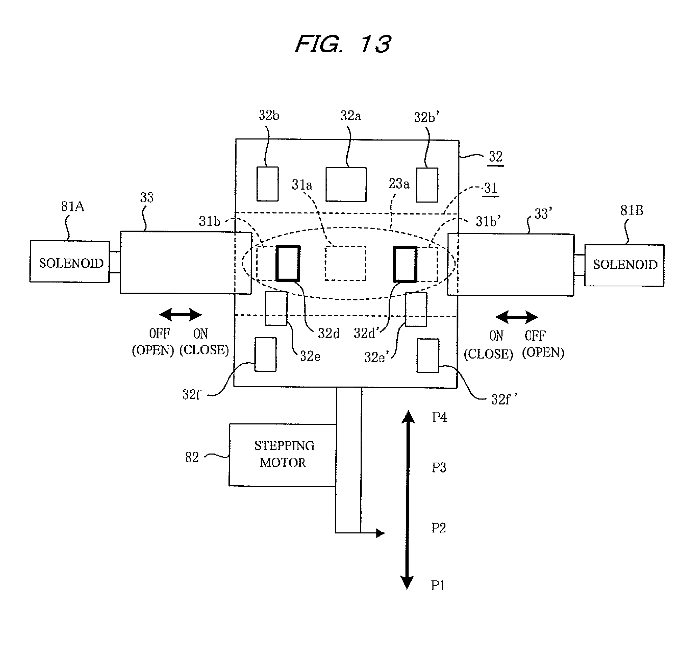

As shown in FIG. 13, the shield plates 33, 33' are moved in a reciprocating manner to the closed position for closing and blocking off light from the left and right stops 31b, 31b' of the fixed stop plate 31 and to the open position for opening (not blocking off light) the left and right stops 31b, 31b' by driving (switching on and off) the solenoid 81A and the solenoid 81B in the same manner as in Embodiment 1.

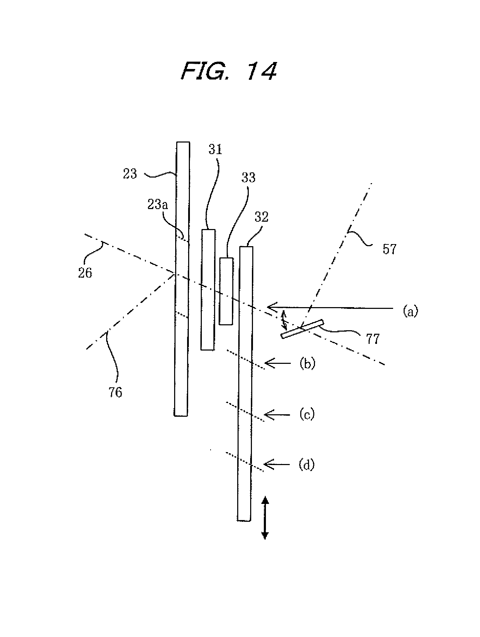

A stepping motor 82 that is driven by the controller 65 moves the movable stop plate 32 in a reciprocating manner between the positions P1 to P4. Position P1 is a position in which the longitudinal position (a) in the movable stop plate 32 in FIG. 12 substantially coincides with the photographic optical axis 26, as shown in FIG. 14. In position P1, the center stop 32a and the FD-light windows 32b, 32b' are effective apertures. In position P2, position (b) of the movable stop plate 32 substantially coincides with the photographic optical axis 26, and the stereo-stops 32d, 32d' are effective apertures. In position P3, position (c) of the movable stop plate 32 substantially coincides with the photographic optical axis 26, and the stereo-stops 32e, 32e' are effective apertures. In position P4, position (d) of the movable stop plate 32 substantially coincides with the photographic optical axis 26, and the stereo-stops 32f, 32f' are effective apertures.

In a state in which the movable stop plate 32 has moved to any of the positions P1 to P4, the solenoids 81A, 81B are independently switched on or off, and the shield plates 33, 33' are moved to the closed position or the open position. This allows the FD-light windows 32b, 32b' as well as the two apertures of each pair of stereo-stops 32d and 32d', 32e and 32e', or 32f and 32f' to be optically blocked off or opened independently from each other.

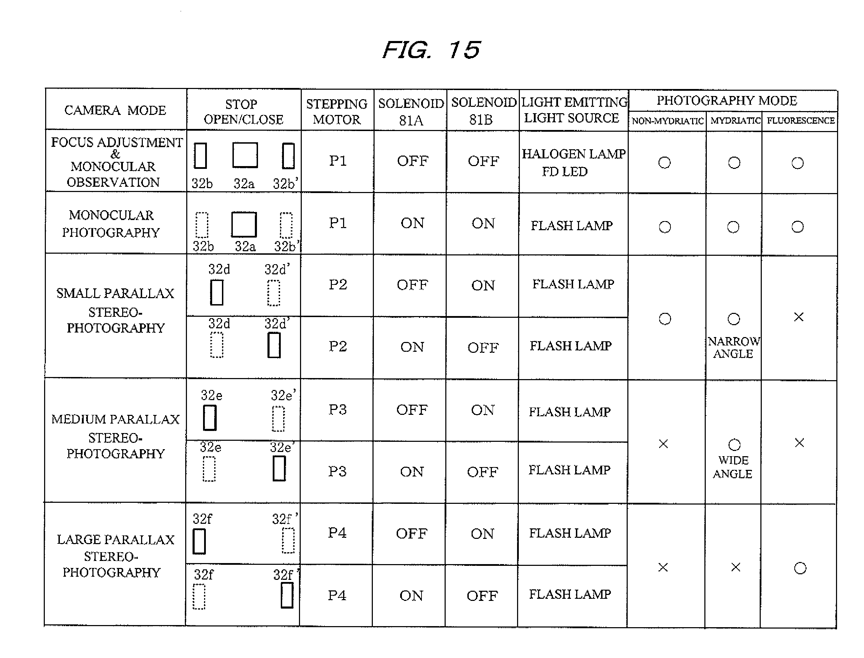

FIG. 15 shows the switching state of the stop apertures in each photography mode. The center stop 32a and the FD-light windows 32b, 32b' are selected as effective apertures during focus adjustment and monocular observation in any of the non-mydriatic, mydriatic, and fluorescence photography modes, and the center stop 32a is selected as the effective aperture in monocular photography. In stereo-photography, the small-parallax stereo-stop 32d or 32d' is selected in non-mydriatic photography; the small-parallax stereo-stop 32d or 32d' is selected in the case of mydriatic photography and a narrow-angle variable lens; and the medium-parallax stereo-stop 32e or 32e' is selected in the case of a wide-angle variable lens. Also, the large-parallax stereo-stop 32f or 32f' is selected when the fluorescence photography mode is used.

Since the shield plates 33, 33' are moved by driving the solenoids 81A, 81B in order to carry out the switching between the left and right stereo-stops, the switching can be carried out at high speed in the same manner as in Embodiment 1.

In Embodiment 1, the FD light is projected into the subject's eye via the FD-light windows 32b, 32b' using two mirrors provided to the upper part of a U-shaped member, but it is possible to instead use a return mirror 77 in the manner shown in FIG. 14. The return mirror 77 is inserted into the photographic optical path when the movable stop plate 32 is in position P1 where the height of position (a) substantially coincides with the photographic optical axis 26. The return mirror 77 is removed from the photographic optical path in conjunction with the movement of the movable stop plate 32 from position P1 to any of the positions P2 to P4.

In accordance with the present embodiment as described above, a suitable aperture can be set for observation and for photography when stereo-photography is carried out. The apparatus of the present embodiment can be variably set to any of the three distances between pupils, i.e., small, medium, and large, by selecting and using any of the three sets of photographic stops 32d and 32d', 32e and 32e', and 32f and 32f' in stereo-photography. This ensures the following advantages.
1) The advantage that the interpupillary distance can be changed depending on the angle of view
   An image having a viewing angle of, e.g., 50° has a greater ocular fundus range displayed on the monitor than an image having a viewing angle of 25° . However, when a photograph has been taken using the same interpupillary distance, there is a problem in that depth perception cannot be obtained in a 50° image, missing information related to small convexities and concavities of the retina. In contrast, a 25° image provides depth perception for small convexities and concavities, so such details are not liable to be missed. However, since the observation range is narrow, there is a problem in that the entire image cannot be ascertained for a macular patient or the like having smooth convexities and concavities.
   These two problems can be overcome by changing the interpupillary distance for 25° photography and 50° photography. In other words, the problems can be overcome by, e.g., increasing the interpupillary distance for 50° in comparison with 25° . However, flare more readily occurs at 50° , so there is demerit in that flare more readily occurs as the interpupillary distance is increased.
2) The advantage that the interpupillary distance can be changed for color photography and fluorescence photography
   Extending the interpupillary distance is directly advantageous for stereographic viewing because the parallax is increased. However, flare more readily occurs when the interpupillary distance is increased. In the case of fluorescence photography, however, flare does not occur in principle because the wavelength of the excitation light is cut by the barrier filter. Accordingly, the interpupillary distance can be extended without concern for flare in fluorescence photography. For this reason, the interpupillary distance can be increased for stereographic viewing in FAG photography in comparison with the color photography mode.
3) The advantage that the interpupillary distance can be changed for mydriatic mode and non-mydriatic mode

There is a need to be able to photograph a pupil diameter of, e.g., 5.5 mm in the mydriatic mode, and 4.0 mm in the non-mydriatic mode. The most effective combination between the possible amount of photographic light and the stereo parallax can accordingly be obtained by varying the interpupillary distance in stereo-photography. For example, the best setting is thought to be an interpupillary distance of 3 mm when the photographable pupil diameter is 5.5 mm, and an interpupillary distance of 2 mm when the photographable pupil diameter is 4 mm. In FIG. 15, the interpupillary distance is varied from the non-mydriatic mode only when a wide angle is used in the mydriatic mode.

The arrangement of the apertures of each of the photographic stops of the movable stop plate 32 shown in FIG. 12 of the present embodiment may be modified in the following manner.

First, a plurality of sizes can be prepared for the center stop 32a that is selected during observation and in monocular photography, and the sizes can be made to correspond to small-pupil photography, or to high-magnification (narrow angle) photography in fluorescent light.

Since movement from position (a) to another position is best carried out as rapidly as possible, position (a) may be disposed between the (b) and (c) positions, or between the (c) and (d) positions.

Specifically, the following arrangement is possible in sequence from the top of the movable stop plate 32. A smaller center stop than the center stop of position (a) is disposed in the position (a1), and a larger center stop than the center stop of position (a) is disposed in the position (a2).
Position (a1): in which the stops for observation and monocular photography are arranged in the case of non-mydriatic and small-pupil diameter.
Position (b): in which the stops for small-parallax stereo-photography are arranged.
Position (a): in which the stops for ordinary observation and monocular photography are arranged.
Position (c): in which the stops for medium-parallax stereo-photography are arranged.
Position (a2): in which the stops for observation and monocular photography in fluorescent light are arranged.
Position (d): in which the stops for large-parallax stereo-photography in fluorescent light are arranged.

In Embodiment 2 described above as well, a flare check can be made for the left and/or the right image, and stereo-photography can be carried out in the selected stereo-stops 32d and 32d', 32e and 32d', or 32f and 32f' in the same manner as in Embodiment 1.

When stereo-photography, three consecutive images, or other consecutive shots can be taken, the flash intensity established during capturing of the first and second images can be adjusted and the brightness of the two images can be made to be substantially the same in the same manner as in Embodiment 1. Also, the consecutive-shot time interval can be adjusted in accordance with the photography mode or the imaging means.

In Embodiments 1 and 2, push-pull solenoids are used as the solenoids 81A to 81C for moving the shield plates 33, 33', but rotary solenoids may also be used instead. In this case, the shield plate may be moved via a mechanism for converting the rotation of the rotary solenoid to a movement of the shield plate in the lateral direction.

In Embodiments 1 and 2, the open/closed state of the two stereo-stops (32d, 32d'; 32e, 32e'; 32f, 32f') is preferably initially set depending on the photography mode. For example, the initial setting may be one in which the two apertures of the stereo-stops are open when the non-mydriatic photography mode has been selected, and any one of the apertures is opened when the mydriatic photography mode has been selected.

## Claims

1. An ophthalmic photography apparatus comprising:
imaging means for photographing a subject's eye as an electronic image via a photographic stop;
recording means for recording the image of the photographed subject's eye; and
a switching mechanism for moving a first movable plate and a second movable plate to switch an aperture of the photographic stop, wherein
the first movable plate is an aperture-switching plate for switching the aperture of a monocular photographic stop and the aperture of a stereo-photographic stop;
the second movable plate is a movable shield plate for opening or optically blocking off any of a plurality of apertures of the first movable plate; and
a combination of the first movable plate and the second movable plate forms a monocular photographic stop or a stereo-photographic stop.

2. An ophthalmic photography apparatus according to claim 1, wherein the monocular photographic stop is a single aperture, and the stereo-photographic stop is two bilaterally symmetric apertures.

3. An ophthalmic photography apparatus according to claim 1 or 2, wherein the two apertures of the stereo-photographic stop are sequentially switched in a single shutter operation.

4. An ophthalmic photography apparatus according to any of claims 1 through 3, wherein the first movable plate causes the monocular photographic stop to be disposed in a position of the optical axis when it is in a first position, and the two apertures of the stereo-photographic stop to be disposed in a bilaterally symmetric position about the center of the optical axis when it is in a second position.

5. An ophthalmic photography apparatus according to claim 4, wherein the two apertures of the stereo-photographic stop are alternately opened and closed by the movement of the second movable plate when the first movable plate is in the second position.

6. An ophthalmic photography apparatus according to any of claims 1 to 5, wherein the second movable plate comprises movable plates provided separately to each of the two apertures of the stereo-photographic stop.

7. An ophthalmic photography apparatus according to any of claims 4 to 6, wherein two apertures for transmitting focus-marker light for focusing is formed in the first movable plate, and the focus-marker light passes through the two apertures and is projected into the subject's eye when the first movable plate is in the first position.

8. An ophthalmic photography apparatus according to any of claims 1 to 7, wherein a plurality of pairs, each comprising two apertures for opening each of the two apertures of the stereo-photographic stop, is formed at different distances between the two apertures in the first movable plate, and any of the aperture pairs is selected to vary the interpupillary distance in stereo-photography.

9. An ophthalmic photography apparatus according to any of claims 1 to 8, wherein a long hole for accommodating an optical fiber for projecting alignment-marker light is formed in the first movable plate along the movement direction of the first movable plate.

10. An ophthalmic photography apparatus according to any of claims 2 to 9, wherein opening and closing of the two apertures of the stereo-photographic stop can be switched during observation or during focus adjustment carried out prior to photography.

11. An ophthalmic photography apparatus according to any of claims 2 to 10, wherein the opening and closing of the two apertures of the stereo-photographic stop in stereo-photography is determined in accordance with the state of the apertures of the photographic stop immediately prior to the shutter operation.
